# EUROPEAN PATENT SPECIFICATION

(11) **EP 1 681 053 B1**
(45) Date of publication and mention of the grant of the patent: **08.10.2014**
(21) Application number: 04793187.8
(22) Date of filing: 22.10.2004
(51) Int. Cl.: A61K 9/16, A61K 31/09, A61P 39/06

(54) **Composition containing clathrated reduced coenzyme Q**
Zusammensetzung mit reduziertem Coenzym Q als Clathrat
Composition contenant une coenzyme Q réduite sous forme de clathrate

(30) Priority: 31.10.2003 JP 2003371793
(43) Date of publication of application: 19.07.2006
(73) Proprietor: KANEKA CORPORATION, Osaka (JP)
(72) Inventor: TANAKA, Hozumi, Kobe-shi, Hyogo 6580015 (JP); FUJII, Kenji, Kobe-shi, Hyogo 6511202 (JP); KAWABE, Taizo, Takasago-shi, Hyogo 6760082 (JP)
(74) Representative: HOFFMANN EITLE
(86) International application number: PCT/JP2004/016079
(87) International publication number: WO 2005/041945

(56) References cited:
- EP-A1- 1 440 962
- EP-A2- 0 519 428
- JP-A- 5 178 765
- JP-A- 57 021 319
- JP-A- 63 083 021
- JP-A- 2002 104 922
- JP-A- 2003 119 126
- JP-A- 2003 520 827
- US-A1- 2003 003 122

## Description

### TECHNICAL FIELD

This invention relates to the provision, as a material for use in foods, functional foods, drugs or quasidrugs, of a reduced coenzyme Q-containing composition which is excellent in solubility or dispersibility in water and in storage stability in spite of the nonuse of any of those natural emulsifiers or synthetic emulsifiers and/or liposomes which are in general and frequent use.

### BACKGROUND ART

Coenzymes Q are essential constituents widely distributed in living bodies, from bacteria to mammals and are known as mitochondrial electron transfer system constituents in cells of living bodies. Through repeated oxidation and reduction in mitochondria, coenzymes Q perform their function as transmitter components in the electron transfer system and, further, reduced coenzymes Q are known to have antioxidant activity. In humans, coenzyme Q₁₀, whose coenzyme Q side chain comprises 10 repeating structures, is the main component and, generally, about 40 to 90% thereof occurs in reduced form in living bodies. The physiological activities of coenzymes Q may be energy production activation through mitochondrial activation, cardiac function activation, cell membrane stabilizing effect, and cell protecting effect through antioxidant activity.

Coenzymes Q are known to be useful in various application fields. For example, oxidized coenzyme Q₁₀ is used as a remedy for congestive heart failure owing to its effects on the heart. Besides such medical uses, they are orally used as nutrients or nutritional supplements, like vitamins, in the form of supplements or health drinks.

In recent years, various reports have been published about the aggravation of diseases due to increases in oxidative stress in blood. Typical examples are arteriosclerosis, complications of diabetes and the like diseases. These diseases are caused and/or aggravated by denaturation of lipids and the like due to various oxidative stresses occurring in blood. For counteracting such effects of oxidative stresses, antioxidant activity promotion in living bodies by administration of an antioxidant is effective. Vitamin E is a compound representative of the liposoluble antioxidants considered to be more effective in inhibiting lipid peroxidation and is in wide use as antioxidants in disease prevention and so on. On the other hand, it has been reported that the coexistence of reduced coenzyme Q₁₀ is important for vitamin E to properly perform its antioxidant activity (Bowry et al., 1993, J. American Chemical Society, 115, 6029-6044), and the importance of coenzymes Q, together with vitamin E, as liposoluble antioxidant substances is becoming clear.

Coenzymes Q have themselves strong antioxidant activity and, therefore, the antioxidant activity in blood can be effectively enhanced by sending a sufficient amount of reduced coenzymes Q in solubilized form into blood.

The enhanced antioxidant activity in blood is considered to be useful widely in preventing vascular lesions during ischemia-reperfusion, preventing restenosis in arteriosclerosis, preventing vascular lesions following cerebral infarction, preventing arteriosclerosis, preventing complications of diabetes, and preventing a number of other diseases from being aggravated supposedly by active oxygen species.

Furthermore, by sending it into the living body in a new delivery form, namely by drip, it becomes possible to provide patients with a serious illness or a brain disease, who are incapable of oral intake, with coenzymes Q. It is thus expected that solubilization of coenzymes Q will bring about a number of merits.

As is well known, coenzymes Q can occur in both the oxidized form and reduced form, and a number of investigations have so far been made about the method for solubilizing oxidized coenzyme Q₁₀ (also called as ubidecarenone or ubiquinone). As for the solubilization of oxidized coenzyme Q₁₀, various methods have been reported, for example coating with liposomes, suspension using a surfactant or an oil and fat, and the like (for example Japanese Kokai Publication Hei-05-186340, Japanese Kokai Publication Hei-07-69874, Japanese Kohyo Publication 2000-510841).

It is necessary for oxidized coenzyme Q₁₀ to be converted to reduced coenzyme Q₁₀ by the action of a reductase or the like in order to perform its antioxidant activity. Supposedly, oxidized coenzyme Q₁₀ taken orally is converted in vivo to reduced coenzyme Q₁₀ by means of NADPH-CoQ reductase, lipoamide dehydrogenase, thioredoxin reductase or the like to show antioxidant activity. However, there is also a fear of decrease, with aging, in the ability of such reductases and other enzymes occurring in vivo to reduce oxidized coenzyme Q₁₀ to reduced coenzyme Q₁₀. Therefore, it is of significance to administer reduced coenzyme Q₁₀ from outside the body to the aged or those showing decreased oxidized coenzyme Q₁₀ reducing activity.

Reduced coenzyme Q₁₀ itself has antioxidant activity, hence it is a substance much expected to be of great utility in the prevention/treatment of such diseases as mentioned above. However, reduced coenzyme Q₁₀ is an extremely unstable substance with respect to its physical property, and is auto-oxidized when it is exposed to air and moreover hardly dissolves in water. Therefore, reduced coenzyme Q₁₀ has not been put into industrial use before.

Although a search report describing the preparation of reduced coenzyme Q₁₀ as a liposome-coated preparation for the purpose of studying oxidoreductases and the like is available (Kishi et al., 1999, BioFactors, 10, 131-138), the liposomes used were prepared extemporaneously in each experiment. Any method for stably solubilizing reduced coenzymes Q has not been known at all.

As a result of the intensive investigations previously made by the inventors, methods for preparing and storing liquid compositions in which reduced coenzyme Q₁₀ solubilized in water can be stably maintained could be found out (Japanese Kokai Publication 2003-026625, Japanese Kokai Publication 2003-119126, EP 1440962 A1). These inventions have made it possible to provide highly useful liquid compositions containing a reduced coenzyme Q solubilized in water to practical use. The present invention is an extension to those previous inventions and has the object to solubilize reduced coenzyme Q₁₀ in water and, at the same time, further improve the storage stability thereof.

### SUMMARY OF THE INVENTION

It is an object of the present invention to provide a composition containing a reduced coenzyme Q, which is a compound very unstable and poor in solubility or dispersibility in water, showing good solubility or dispersibility in water and capable of being stored at room temperature for a prolonged period of time.

The present inventors made intensive investigations to accomplish the above object and, as a result, found that the coexistence of a reduced coenzyme Q with a cyclodextrin results in marked improvement in storage stability and at the same time gives a composition having good dispersibility in water in spite of the fact that the reduced coenzyme Q is physically very unstable and almost insoluble in water. Such findings have now led to completion of the present invention.

Thus, the present invention relates to a composition containing a cyclodextrin, a polar solvent and a reduced coenzyme Q represented by the general formula (1); 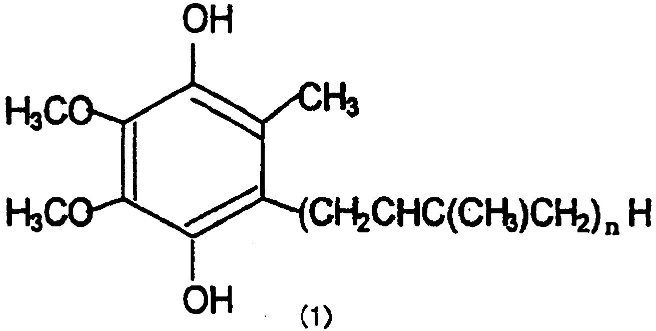 (in the formula, n is an integer of 1 to 12); wherein the reduced coenzyme Q is clathrated by the cyclodextrin and solubilized in said composition.

Further, the invention relates to a method for solubilizing a reduced coenzyme Q which comprises mixing a cyclodextrin, a polar solvent and a reduced coenzyme Q represented by the formula (1) given above to give a clathrate compound.

The invention also relates to a method for producing a composition containing a cyclodextrin, a polar solvent and a reduced coenzyme Q, wherein the reduced coenzyme Q is solubilized in said composition, which method comprises mixing the cyclodextrin, the polar solvent and the coenzyme Q represented by the general formula (1) given above to give a clathrate compound.

The invention further relates to a method for stabilizing a reduced coenzyme Q which comprises mixing a cyclodextrin, a polar solvent and a reduced coenzyme Q represented by the general formula (1) given above to give a clathrate compound.

The invention still further relates to a method for inhibiting the oxidation of a reduced coenzyme Q which comprises mixing a cyclodextrin, a polar solvent and the reduced coenzyme Q represented by the general formula (1) given above to give a clathrate compound.

### DETAILED DESCRIPTION OF THE INVENTION

First, the composition of the invention which contains a cyclodextrin, a polar solvent and a reduced coenzyme Q, wherein the reduced coenzyme Q is solubilized in said composition, is described. The term "solubilized" is used herein to indicate that the coenzyme Q in question is dissolved in the composition mentioned above and the whole or a part of the coenzyme Q is clathrated in the cyclodextrin. The above composition may contain the part of the coenzyme Q which remains undissolved in the composition but is dispersed therein. The term "dispersed" is used herein to indicate that when the composition is subjected to the test described below, the coenzyme Q in the composition neither rises to the surface nor precipitates. The term "clathrated" is used herein to indicate a state of association of molecules constituting the composition so as to allow the coenzyme Q-containing composition to dissolve in water.

Whether a reduced coenzyme Q is solubilized in the above composition or not can be judged by the following test. Thus, 1 g of the composition is suspended in 500 ml of water and, after a lapse of 24 hours at 20°C, the suspension is observed by the eye. When a state of uniform dispersion can be confirmed, it is judged that the reduced coenzyme Q is in a solubilized state. Whether a reduced coenzyme Q is clathrated in a cyclodextrin or not can be judged by the following test. Thus, 1 g of the composition is suspended in 500 ml of water and, after a lapse of 24 hours at 20°C, the suspension is observed by the eye. When a state of uniform dispersion and a transparent solution state can be confirmed, it is judged that the reduced coenzyme Q is clathrated in the cyclodextrin.

Coenzymes Q are represented by the formula (1); 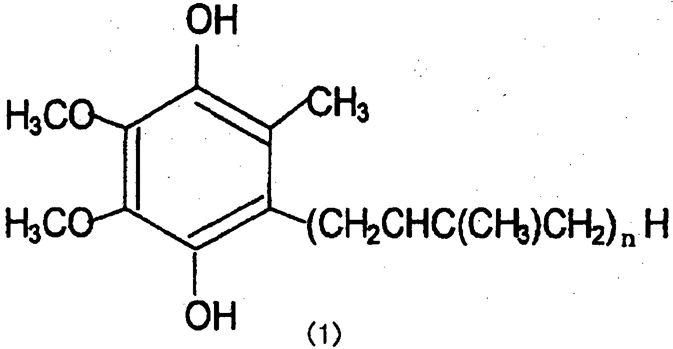 (in the formula, n represents an integer of 1 to 12); and by the formula (2); 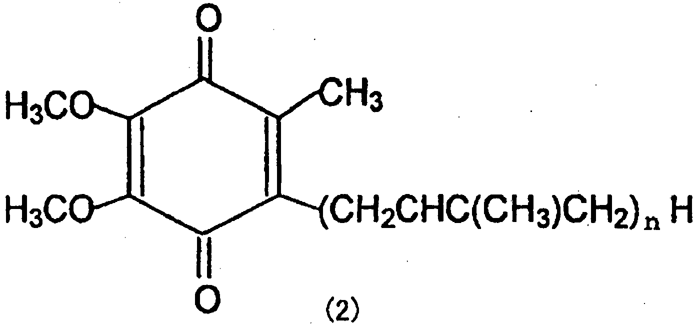 (in the formula, n represents an integer of 1 to 12); and the formula (1) represents reduced coenzymes Q and the formula (2) represents oxidized coenzymes Q. A reduced coenzyme Q may be crystalline or noncrystalline.

The composition of the invention may contain an oxidized coenzyme Q. When it contains both an oxidized coenzyme Q and a reduced coenzyme Q, the proportion of the reduced coenzyme Q to the total amount of the coenzymes Q (the sum of the oxidized coenzyme Q and the reduced coenzyme Q) is preferably not smaller than 50% by weight, more preferably not smaller than 75% by weight.

The method for preparing the reduced coenzyme Q is not particularly restricted but, for example, the method comprising producing a coenzyme Q by any of the methods known in the art, for example by synthesis, fermentation, extraction from a natural source, and then concentrating the reduced coenzyme Q fraction in the eluate by chromatography can be employed. In this case, it is also possible to add a conventional reducing agent, such as sodium borohydride or sodium dithionite (hydrosulfite sodium), to the coenzyme Q if necessary and, after reduction of the oxidized coenzyme Q contained in the above coenzyme Q to a reduced coenzyme Q in the conventional manner, subject the reduction product to chromatography for concentration. It can also be obtained by the method comprising reacting a commercially available high-purity coenzyme Q with such a reducing agent as mentioned above.

The reduced coenzyme Q to be used in the present invention may be any of those in which the number (n in the formulas) of repeating units in the side chain is 1 to 12, as represented by the above formulas (1) and (2). However, the one having 10 repeating units in the side chain, namely reduced coenzyme Q₁₀, can be used most adequately of all.

Cyclodextrin is not particularly restricted but, for example, α-cyclodextrin, β-cyclodextrin, γ-cyclodextrin can be used. Preferably the one selected from α-cyclodextrin and γ-cyclodextrin is used. More preferably γ-cyclodextrin is used.

A proportion of a cyclodextrin contained is preferably 0.1 to 100 mole, and more preferably 0.2 to 20 mole, per 1 mole of a reduced coenzyme Q.

The term "polar solvent" is used herein to indicate water or alcohols. The polar solvent contained is not particularly restricted but includes water and alcohols such as ethanol. These may be used singly or in combination thereof. A mixed solution containing water and ethanol is preferably used and water is more preferably used.

A proportion of a reduced coenzyme Q to a polar solvent is not particularly restricted. However, the usage of a reduced coenzyme Q to a polar solvent is usually 0.001 to 500 % by weight.

The composition of the invention which contains a reduced coenzyme Q in a solubilized form may contain an antioxidant. The antioxidant may be any compound having antioxidant activity and preferably comprises one or a mixture of two or more selected from citric acid, citric acid derivatives, vitamin C (ascorbic acid), vitamin C derivatives, vitamin E, vitamin E derivatives, glutathione, reduced glutathione, sodium thiosulfate, L-cysteine, L-carnitine, lycopene, riboflavin, curcuminoids, superoxide dismutase (SOD) and PQQ (pyrroloquinoline quinine). Furthermore, one or a mixture of two or more selected from citric acid, citric acid derivatives, vitamin C (ascorbic acid), vitamin C derivatives, vitamin E and vitamin E derivatives is more preferably used. The antioxidant to be contained is used preferably in an amount of not smaller than 0.01 mole but not greater than 1000 moles, more preferably not smaller than 0.1 mole but not greater than 100 moles, per 1 mole of a reduced coenzyme Q.

Now, a method for solubilizing a reduced coenzyme Q according to the invention is described. As the method of solubilization, there may be mentioned, for example, the method comprising bringing a reduced coenzyme Q into contact with a cyclodextrin, or mixing them together, in an appropriate polar solvent. Upon such contacting or mixing, a clathrate compound can be obtained. On the occasion of contacting or mixing, vigorous stirring of raw materials is preferred from the viewpoint of reduction of the time required for clathrate formation. This is made for securing sufficient contact between a reduced coenzyme Q and a cyclodextrin. The conditions of vigorous stirring so referred to herein are, for example, as follows: 10 minutes of treatment at room temperature (about 20°C) at 20,000 rpm using a homomixer (Polytron PT2100).

As the polar solvent, there may be mentioned the same ones as mentioned hereinabove for the composition, for example, water and alcohols such as ethanol. These may be used in admixture. A mixture of water and ethanol is preferred, and water is more preferred. A reduced coenzyme Q may be used as such or in the form of a solution in a nonpolar solvent or a fat or oil.

Usable as the nonpolar solvent are, for example, hexane, ethers, and oils and fats. Preferred as the oils and fats are olive oil, perilla oil, rapeseed oil, rice bran oil, soybean oil, fish oil since they are edible. These may be used in combination. The cyclodextrin may be used in the form of a solution in a polar solvent or as such.

The order of mixing the reduced coenzyme Q, the cyclodextrin and the polar solvent is not particularly restricted. Preferably, however, the cyclodextrin is dissolved in the polar solvent, and the reduced coenzyme Q is then added to the solution for contacting and mixing up. More preferred is a procedure comprising dissolving the cyclodextrin in the polar solvent and simultaneously adding the reduced coenzyme Q for contacting and mixing up. In these procedures, the reduced coenzyme Q is clathrated by the cyclodextrin and a clathrate compound is formed. In this way, a composition containing a reduced coenzyme Q solubilized and soluble or uniformly dispersible in water is obtained.

The order of mixing the reduced coenzyme Q, the cyclodextrin, the polar solvent and the antioxidant is not particularly restricted. For example, it is possible to dissolve the antioxidant in the polar solvent, add the cyclodextrin to the solution and, thereafter, add the reduced coenzyme Q for contacting and mixing up. Alternatively, it is also possible to add the cyclodextrin to the polar solvent, then add the reduced coenzyme Q for contacting and mixing up and, thereafter, add the antioxidant. The antioxidant may be used either as such or in the form of a solution in the above-mentioned polar solvent.

The compositions containing a reduced coenzyme Q solubilized by the method of solubilization all fall within the scope of the present invention, as defined by the claims.

A further description is now given of the method for producing a composition containing a cyclodextrin, a polar solvent and a reduced coenzyme Q represented by the formula (1) given hereinabove, wherein the reduced coenzyme Q is solubilized therein, which method comprises mixing the cyclodextrin, the polar solvent and the reduced coenzyme Q. In carrying out that method, a proportion between a reduced coenzyme Q and an oxidized coenzyme Q, other materials to be used, an order of feeding of materials, a proportion between a reduced coenzyme Q and a cyclodextrin and other conditions are the same as those described above referring to the method of solubilization.

The composition according to the invention can be subjected to spray drying, freeze-drying to give a powdery composition. More preferably, the composition is to be obtained in the form of a powdery composition in view of the simplicity in practical handling and the ease of industrial utilization. In this way, a composition excellent in solubility or dispersibility in water and capable of being stored at room temperature or in a refrigerator for a long period of time can suitably be obtained.

The composition to be prepared may contain one or more of other pharmaceutically acceptable materials. The pharmaceutical materials that can be contained in the composition include, for example, colorants, emulsifiers, tonicity adjusting agents, buffering agents, dissolution aids, preservatives, correctives, stabilizers, excipients. The composition may further contain one or more of other active ingredients, for example, drugs, nutrient supplements, according to the intended use thereof. These pharmaceutical materials can be properly prepared in the conventional manner. The composition of the invention can be further subjected to granulation, for example, extrusion granulation, fluidized bed granulation.

Further, the composition may contain one or more of various ingredients considered to be useful in maintaining the health of animals, including humans, pets, domestic animals, for example, medicinal ingredients, functional food components, supplement components or the like. These ingredients can be properly added in the conventional manner.

A dosage form of the composition obtained in accordance with the invention is not particularly restricted but can be adequately selected according to the intended use thereof. In view of its good solubility or dispersibility in water, it is preferably used for oral administration.

The reduced coenzyme Q-containing composition of the invention can be used in foods, functional foods, drugs, quasidrugs for humans or animals. The term "functional foods" is used herein to indicate foods to be taken for health maintenance or for nutrition in lieu of taking meals, for example, health foods, dietary supplements, supplements, nutritious foods. More specific forms include, health drinks, chewable tablets, capsules, tablets, injections, transfusions, ophthalmic solutions, feeds.

The solubilized composition obtained by the solubilizing method of the invention can stably maintain a reduced coenzyme Q against oxidation thereof. The term "can stably maintain" is used herein to indicate that when the solubilized composition is stored, in a state protected against light, in an air atmosphere at room temperature (about 20ºC) for 4 weeks, the residual reduced coenzyme Q percentage as evaluated is not lower than 80% relative to the concentration at the time of starting storage, although the limit residual percentage may vary depending on the intended use or purpose of the composition and on the storage conditions.

Now, a description is given of the method for preventing the oxidation of a reduced coenzyme Q according to the invention which method comprises mixing a cyclodextrin, a polar solvent and a reduced coenzyme Q represented by the formula (1) given hereinabove. After 4 weeks of storage of a reduced coenzyme Q, in a state protected against light, in an air atmosphere at room temperature (about 20ºC), when the residual reduced coenzyme Q percentage is not lower than 80% of the amount at the time of starting storage, the oxidation of the reduced coenzyme Q is judged to have been inhibited according to the method. In carrying out the method, such conditions as a proportion between a reduced coenzyme Q and an oxidized coenzyme Q, other materials to be used, and a proportion between a reduced coenzyme Q and a cyclodextrin are the same as in the case of the composition described above.

### BRIEF DESCRIPTION OF THE DRAWINGS

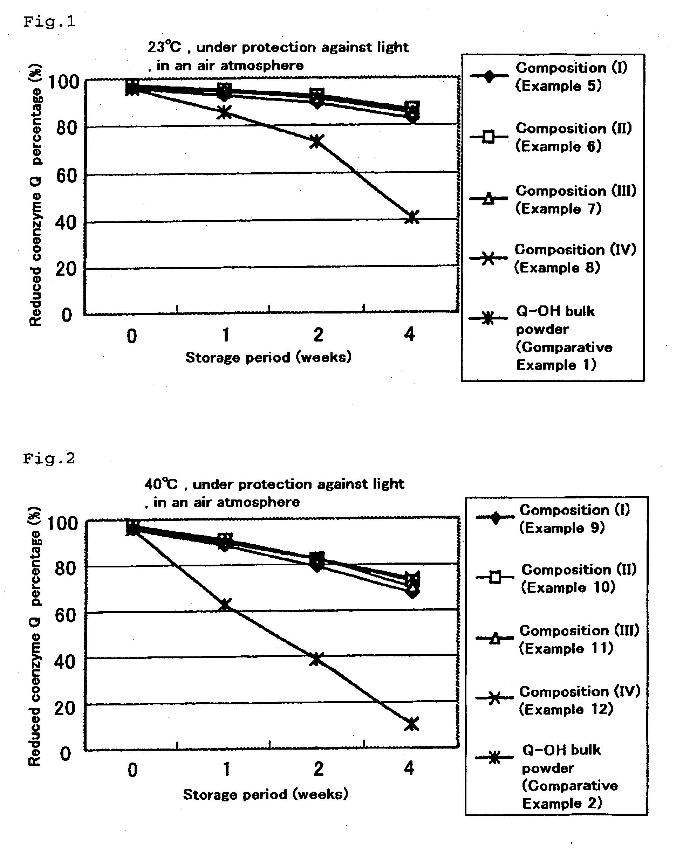

Fig. 1 is a graphic representation of the stability of reduced coenzyme Q₁₀ in each powdery composition against autooxidation at 23ºC. The ordinate denotes residual reduced coenzyme Q₁₀ percentage relative to total coenzyme Q₁₀ contained in the composition (test sample), and the abscissa denotes the number of storage days. At 4 weeks after starting storage, the bulk powder of reduced coenzyme Q₁₀ showed a decrease in the reduced coenzyme Q percentage to 41% whereas the reduced coenzyme Q percentage was not lower than 80% in all the powder compositions I-IV.
Fig. 2 is a graphic representation of the stability of reduced coenzyme Q₁₀ in each powdery composition against autooxidation at 40ºC. The ordinate denotes the residual reduced coenzyme Q₁₀ percentage relative to the total coenzyme Q₁₀ contained in the composition (test sample), and the abscissa denotes the number of storage days. At 4 weeks after starting storage, the bulk powder of reduced coenzyme Q₁₀ showed a decrease in the reduced coenzyme Q percentage to 11% whereas the reduced coenzyme Q percentage was not lower than 65% in all the powder compositions I-IV.

### BEST MODES FOR CARRYING OUT THE INVENTION

The following examples elucidate the effects of the invention.

In the examples, a reduced coenzyme Q concentration and an oxidized coenzyme Q concentration were determined by the following HPLC analysis.

### (HPLC analysis conditions)

Column; SYMMETRY C18 (product of Waters Corporation), 250 mm (length), 4.6 mm (inside diameter), mobile phase; C₂H₅OH : CH₃OH = 4:3 (v:v), detection wavelength; 210 nm, flow rate; 1 ml/min, retention time for a reduced coenzyme Q₁₀; 9.1 min, retention time for an oxidized coenzyme Q₁₀; 13.3 min.

The spray drying was performed at an inlet temperature of 170ºC, an outlet temperature of 80ºC and a spray rate of 100 ml/min using an Okawara Mfg. Co., Ltd. model L-12 spray drier.

### (Example 1)

205 g of γ-cyclodextrin was dissolved in 1336 ml of water, immediately thereafter, 18 g of reduced coenzyme Q₁₀ (containing 2% of oxidized coenzyme Q₁₀) was added, followed by 20 minutes of stirring at 10,000 rpm using a homogenizer so that a mixture of reduced coenzyme Q₁₀ and the cyclodextrin was prepared. The thus-prepared reduced coenzyme Q₁₀-cyclodextrin mixture was subjected to spray drying to give a powdery composition (I). The mole proportions of the materials constituting this powdery composition were; reduced coenzyme Q₁₀ : γ-cyclodextrin = 1:8.

### (Example 2)

12.2 g of ascorbic acid was dissolved in 675 ml of water with thorough stirring, 85.3 g of γ-cyclodextrin was then dissolved in the solution and, immediately thereafter, 15 g of reduced coenzyme Q₁₀ (containing 2% of oxidized coenzyme Q₁₀) was added, followed by 20 minutes of stirring at 10,000 rpm using a homogenizer so that a mixture of reduced coenzyme Q₁₀ and the cyclodextrin was prepared. The thus-prepared reduced coenzyme Q₁₀-cyclodextrin mixture was subjected to spray drying to give a powdery composition (II). The mole proportions of the materials constituting this powdery composition were; reduced coenzyme Q₁₀ : γ-cyclodextrin : ascorbic acid = 1:4:4.

### (Example 3)

12.2 g of ascorbic acid was dissolved in 1188 ml of water with thorough stirring, 171 g of γ-cyclodextrin was then dissolved in the solution and, immediately thereafter, 15 g of reduced coenzyme Q₁₀ (containing 2% of oxidized coenzyme Q₁₀) was added, followed by 20 minutes of stirring at 10,000 rpm using a homogenizer so that a mixture of reduced coenzyme Q₁₀ and the cyclodextrin was prepared. The thus-prepared reduced coenzyme Q₁₀-cyclodextrin mixture was subjected to spray drying to give a powdery composition (III). The mole proportions of the materials constituting this powdery composition were; reduced coenzyme Q₁₀ : γ-cyclodextrin : ascorbic acid = 1:8:4.

### (Example 4)

16.3 g of ascorbic acid was dissolved in 1182 ml of water with thorough stirring, 171 g of γ-cyclodextrin was then dissolved in the solution and, immediately thereafter, 10 g of reduced coenzyme Q₁₀ (containing 2% of oxidized coenzyme Q₁₀) was added, followed by 20 minutes of stirring at 10,000 rpm using a homogenizer so that a mixture of reduced coenzyme Q₁₀ and the cyclodextrin was prepared. The thus-prepared reduced coenzyme Q₁₀-cyclodextrin mixture was subjected to spray drying to give a powdery composition (IV). The mole proportions of the materials constituting this powdery composition were: reduced coenzyme Q₁₀ : γ-cyclodextrin : ascorbic acid = 1:12:8.

### (Example 5 to 12, Comparative Examples 1 and 2)

Reduced coenzyme Q₁₀ bulk powder (Q-OH bulk powder) containing 2% of oxidized coenzyme Q₁₀ and the powdery compositions I to IV respectively obtained in Examples 1 to 4 were subjected to stability testing in the following manner. Thus, each composition (test sample) was placed in a microtube and stored, in a state protected against light, at 23ºC or 40ºC in the presence of air. The test samples used and the test temperatures are shown in Table 1.

**Table 1**

| | Test samples | Test temperature |
|---|---|---|
| Example 5 | Powdery composition (I) | 23°C |
| Example 6 | Powdery composition (II) | 23°C |
| Example 7 | Powdery composition (III) | 23°C |
| Example 8 | Powdery composition (IV) | 23°C |
| Comparative Example 1 | Reduced coenzyme Q₁₀ bulk powder (Q-OH bulk powder) | 23°C |
| Example 9 | Powdery composition (I) | 40°C |
| Example 10 | Powdery composition (II) | 40°C |
| Example 11 | Powdery composition (III) | 40°C |
| Example 12 | Powdery composition (IV) | 40°C |
| Comparative Example 2 | Reduced coenzyme Q₁₀ bulk powder (Q-OH bulk powder) | 40°C |

After 1, 2 or 4 weeks of storage, 25 mg of each sample was sampled and dissolved in ethanol, the concentrations of oxidized coenzyme Q₁₀ and reduced coenzyme Q₁₀ were measured by HPLC and the percentage of reduced coenzyme Q₁₀ in total coenzyme Q₁₀ as contained in the composition (test sample) was determined. The results are shown in Fig. 1 and Fig. 2.

### (Examples 13 to 16, Comparative Example 3)

### Coenzyme Q₁₀ bulk powder (Q-OH bulk powder)

containing 98% of reduced coenzyme Q₁₀ and the powdery compositions obtained in Examples 1 to 4 were subjected to solubility testing in the following manner. Thus, 1 g of each composition (test sample) was added to 100 ml of water, and the mixture was stirred at 5,000 rpm for 30 seconds using a mixer and then allowed to stand. After 1 hour of standing, the condition of the aqueous solution was observed for confirming the solubility in water. The results are shown in Table 2.

**Table 2**

| | Test samples | Solubility in water |
|---|---|---|
| Example 13 | Powdery composition (I) | Soluble, homogeneously dispersed |
| Example 14 | Powdery composition (II) | Soluble, homogeneously dispersed |
| Example 15 | Powdery composition (III) | Soluble, homogeneously dispersed |
| Example 16 | Powdery composition (IV) | Soluble, homogeneously dispersed |
| Comparative Example 3 | Reduced coenzyme Q₁₀ bulk powder (Q-OH bulk powder) | Insoluble |

Coenzyme Q₁₀ bulk powder (Q-OH bulk powder) containing 98% of reduced coenzyme Q₁₀ was insoluble, forming orange-colored oil drops floating on the water. On the contrary, the powdery compositions I to IV obtained in Examples 1 to 4 were all found to be uniformly dispersed in water in a white emulsion-like state.

### INDUSTRIAL APPLICABILITY

The invention has made it possible to provide reduced coenzymes Q highly useful as antioxidants or nutrient supplement components in the form of compositions soluble in water and, at the same time, excellent in storage stability.

## Claims

1. A composition containing a cyclodextrin, a polar solvent and a reduced coenzyme Q represented by the general formula (1) where n is an integer of 1 to 12: 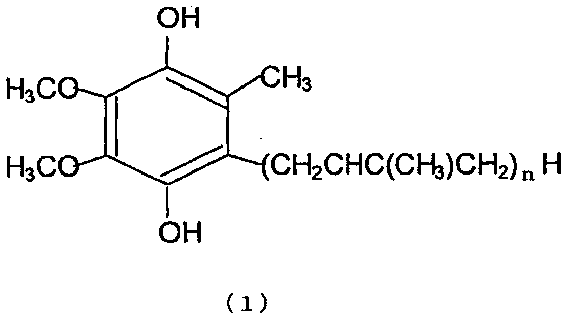 wherein the reduced coenzyme Q is clathrated by the cyclodextrin and the reduced coenzyme Q is solubilized in said composition.

2. The composition according to Claim 1 which further contains an oxidized coenzyme Q, wherein a proportion of the reduced coenzyme Q to the sum of the oxidized coenzyme Q and the reduced coenzyme Q is not smaller than 50% by weight.

3. The composition according to Claim 1which is used for oral administration.

4. The composition according to Claim 1wherein the reduced coenzyme Q is reduced coenzyme Q₁₀.

5. The composition according to Claim 1, wherein the cyclodextrin is γ-cyclodextrin.

6. The composition according to Claim 1wherein the polar solvent is water or a mixed solution of water and an alcohol.

7. The composition according to Claim 1, wherein a proportion of the cyclodextrin contained is 0.1 to 100 mole, per 1 mole of the reduced coenzyme Q.

8. The composition according to Claim 1 which further contains an antioxidant.

9. The solubilized composition according to Claim 8, wherein an antioxidant is at least one species selected from citric acid, citric acid derivatives, vitamin C, vitamin C derivatives, vitamin E, vitamin E derivatives, glutathione, reduced glutathione, sodium thiosulfate, L-cysteine, L-carnitine, lycopene, riboflavin, curcuminoids and superoxide dismutase (SOD).

10. The solubilized composition according to Claim 1 which further contains at least one or more species selected from medicinal ingredients, functional food components, supplement components and food components.

11. A food, functional food, drug or quasidrug for administration to humans or animals which contains the composition according to Claim 1.

12. A powdery solubilized composition which can be obtained by subjecting the composition according to Claim 1 to spray drying.

13. A method for solubilizing a reduced coenzyme Q and inhibiting the oxidation of a reduced coenzyme Q represented by the general formula (1) where n is an integer of 1 to 12: 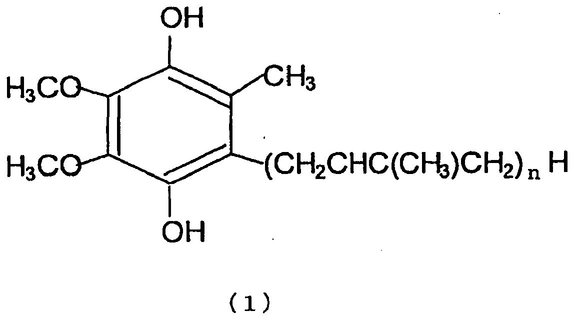 which comprises mixing a cyclodextrin, a polar solvent and the reduced coenzyme Q to give a clathrate compound.

14. The method according to Claim 13, wherein oxidized coenzyme Q is co-present, and wherein a proportion of the reduced coenzyme Q to the sum of the oxidized coenzyme Q and the reduced coenzyme Q is not smaller than 50% by weight.

15. The method according to Claim 13, wherein the reduced coenzyme Q is reduced coenzyme Q₁₀.

16. The method according to Claim 13, wherein the cyclodextrin is γ-cyclodextrin.

17. The method according to Claim 13 which comprises dissolving the cyclodextrin in the polar solvent, and then mixing the reduced coenzyme Q with the obtained solution.

18. The method according to Claim 13, wherein the polar solvent is water or a mixed solution of water and an alcohol.

19. The method according to Claim 13, wherein a proportion of the cyclodextrin contained is 0.1 to 100 mole, per 1 mole of the reduced coenzyme Q.

20. The method according to Claim 13, wherein an antioxidant is co-present, and wherein the antioxidant is at least one species selected from citric acid, citric acid derivatives, vitamin C, vitamin C derivatives, vitamin E, vitamin E derivatives, glutathione, reduced glutathione, sodium thiosulfate, L-cysteine, L-carnitine, lycopene, riboflavin, curcuminoids and superoxide dismutase (SOD).

21. A method for producing a composition containing a cyclodextrin, a polar solvent and a reduced coenzyme Q represented by the general formula (1) where n is an integer of 1 to 12: 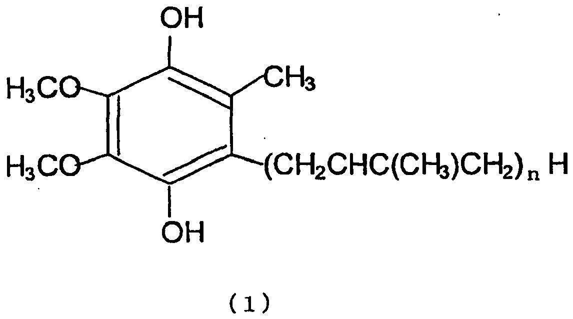 which comprises mixing the reduced coenzyme Q with the cyclodextrin in the polar solvent to give a clathrate compound.

22. The method according to Claim 21 which comprises mixing the cyclodextrin, the polar solvent and the reduced coenzyme Q, and spray drying the mixture to give a powdery composition.

## Patentansprüche

1. Zusammensetzung, die ein Cyclodextrin, ein polares Lösungsmittel und ein reduziertes Co-Enzym Q, das durch die allgemeine Formel (1), in der n eine ganze Zahl von 1 bis 12 ist, dargestellt ist, enthält: 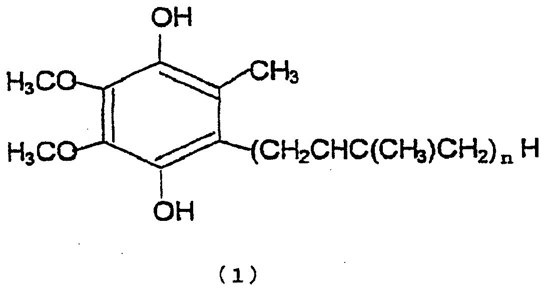 wobei das reduzierte Co-Enzym Q durch das Cyclodextrin eingeschlossen ist und das reduzierte Co-Enzym Q in der Zusammensetzung gelöst ist.

2. Zusammensetzung gemäß Anspruch 1, die ferner ein oxidiertes Co-Enzym Q enthält, wobei das Verhältnis des reduzierten Co-Enzyms Q zu der Summe des oxidierten Co-Enzyms Q und des reduzierten Co-Enzyms Q nicht kleiner als 50 Gew.% ist.

3. Zusammensetzung gemäß Anspruch 1, die für die orale Verabreichung verwendet wird.

4. Zusammensetzung gemäß Anspruch 1, wobei das reduzierte Co-Enzym Q reduziertes Co-Enzym Q₁₀ ist.

5. Zusammensetzung gemäß Anspruch 1, wobei das Cyclodextrin γ-Cyclodextrin ist.

6. Zusammensetzung gemäß Anspruch 1, wobei das polare Lösungsmittel Wasser oder ein gemischtes Lösungsmittel aus Wasser und einem Alkohol ist.

7. Zusammensetzung gemäß Anspruch 1, wobei das Verhältnis des enthaltenen Cyclodextrins 0,1 bis 100 mol pro 1 mol des reduzierten Co-Enzyms Q ist.

8. Zusammensetzung gemäß Anspruch 1, die ferner ein Antioxidationsmittel enthält.

9. Gelöste Zusammensetzung gemäß Anspruch 8, wobei das Antioxidationsmittel mindestens eine Art, ausgewählt aus Zitronensäure, Zitronensäurederivaten, Vitamin C, Vitamin C-Derivaten, Vitamin E, Vitamin E-Derivaten, Glutathion, reduziertem Glutathion, Natriumthiosulfat, L-Cystein, L-Carnitin, Lycopen, Riboflavin, Curcuminoiden und Superoxiddismutase (SOD), ist.

10. Gelöste Zusammensetzung gemäß Anspruch 1, die ferner zumindest eine oder mehrere Arten, ausgewählt aus medizinischen Inhaltsstoffen, funktionellen Nahrungsmittelkomponenten, Ergänzungsmittelkomponenten und Nahrungsmittelkomponenten, enthält.

11. Lebensmittel, funktionelles Lebensmittel, Medikament oder Quasimedikament zur Verabreichung an Menschen oder Tiere, das die Zusammensetzung gemäß Anspruch 1 enthält.

12. Pulverförmige, gelöste Zusammensetzung, die erhalten werden kann, indem die Zusammensetzung gemäß Anspruch 1 einer Sprühtrocknung unterzogen wird.

13. Verfahren zur Lösung eines reduzierten Co-Enzyms Q und zur Verhinderung der Oxidation des reduzierten Co-Enzyms Q, das durch die allgemeine Formel (1), in der n eine ganze Zahl von 1 bis 12 ist, dargestellt ist: 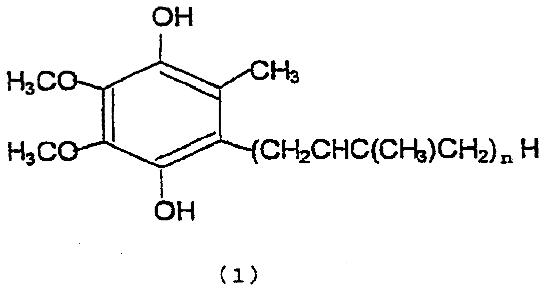 das Mischen von Cyclodextrin, einem polaren Lösungsmittel und dem reduzierten Co-Enzym Q, um eine Clathratverbindung zu erhalten, umfasst.

14. Verfahren gemäß Anspruch 13, worin das oxidierte Co-Enzym Q gleichzeitig vorhanden ist und worin das Verhältnis des reduzierten Co-Enzyms Q zu der Summe des oxidierten Co-Enzyms Q und des reduzierten Co-Enzyms Q nicht kleiner als 50 Gew.% ist.

15. Verfahren gemäß Anspruch 13, worin das reduzierte Co-Enzym Q reduziertes Co-Enzym Q₁₀ ist.

16. Verfahren gemäß Anspruch 13, worin das Cyclodextrin γ-Cyclodextrin ist.

17. Verfahren gemäß Anspruch 13, das das Auflösen des Cyclodextrins in dem polaren Lösungsmittel und dann Mischen des reduzierten Co-Enzyms Q mit der erhaltenen Lösung umfasst.

18. Verfahren gemäß Anspruch 13, worin das polare Lösungsmittel Wasser oder ein gemischtes Lösungsmittel aus Wasser und einem Alkohol ist.

19. Verfahren gemäß Anspruch 13, worin das Verhältnis des enthaltenen Cyclodextrins 0,1 bis 100 mol pro 1 mol des reduzierten Co-Enzyms Q ist.

20. Verfahren gemäß Anspruch 13, worin ein Antioxidationsmittel gleichzeitig vorhanden ist und wobei das Antioxidationsmittel mindestens eine Art, ausgewählt aus Zitronensäure, Zitronensäurederivaten, Vitamin C, Vitamin C-Derivaten, Vitamin E, Vitamin E-Derivaten, Glutathion, reduziertem Glutathion, Natriumthiosulfat, L-Cystein, L-Carnitin, Lycopen, Riboflavin, Curcuminoiden und Superoxiddismutase (SOD), ist.

21. Verfahren zur Herstellung einer Zusammensetzung, die ein Cyclodextrin, ein polares Lösungsmittel und ein reduziertes Co-Enzym Q, das durch die allgemeine Formel (1), in der n eine ganze Zahl von 1 bis 12 ist, dargestellt ist, umfasst: 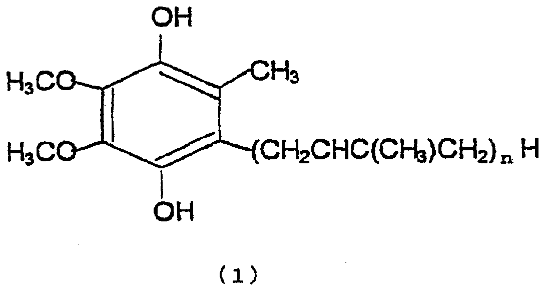 das Mischen des reduzierten Co-Enzyms Q mit dem Cyclodextrin in dem polaren Lösungsmittel, um eine Clathratverbindung zu erhalten, umfasst.

22. Verfahren gemäß Anspruch 21, das Mischen des Cyclodextrins, des polaren Lösungsmittels und des reduzierten Co-Enzyms Q und Spraytrocknen der Mischung, um eine pulverförmige Zusammensetzung zu erhalten, umfasst.

## Revendications

1. Composition contenant une cyclodextrine, un solvant polaire et une coenzyme Q réduite représentée par la formule générale (1) où n est un nombre entier de 1 à 12 : 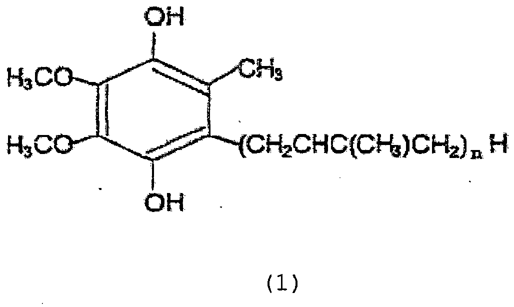 dans laquelle la coenzyme Q réduite est clathratée par la cyclodextrine et la coenzyme Q réduite est solubilisée dans ladite composition.

2. Composition selon la revendication 1, qui contient en outre une coenzyme Q oxydée, dans laquelle une proportion de la coenzyme Q réduite par rapport à la somme de la coenzyme Q oxydée et la coenzyme Q réduite n'est pas inférieure à 50 % en poids.

3. Composition selon la revendication 1 qui est utilisée pour une administration orale.

4. Composition selon la revendication 1, dans laquelle la coenzyme Q réduite est la coenzyme Q₁₀ réduite.

5. Composition selon la revendication 1, dans laquelle la cyclodextrine est la γ-cyclodextrine.

6. Composition selon la revendication 1, dans laquelle le solvant polaire est l'eau ou une solution mixte d'eau et d'un alcool.

7. Composition selon la revendication 1, dans laquelle une proportion de la cyclodextrine contenue est de 0,1 à 100 moles pour 1 mole de la coenzyme Q réduite.

8. Composition selon la revendication 1, qui contient en outre un antioxydant.

9. Composition solubilisée selon la revendication 8, dans laquelle un antioxydant est au moins une espèce choisie parmi l'acide citrique, les dérivés de l'acide citrique, la vitamine C, les dérivés de la vitamine C, la vitamine E, les dérivés de la vitamine E, le glutathion, le glutathion réduit, le thiosulfate de sodium, la L-cystéine, la L-carnitine, le lycopène, la riboflavine, les curcuminoïdes et la superoxyde dismutase (SOD).

10. Composition solubilisée selon la revendication 1, qui contient en outre au moins une ou plusieurs espèces choisies parmi les ingrédients médicinaux, les composants d'aliments fonctionnels, les composants de compléments et les composants alimentaires.

11. Aliment, aliment fonctionnel, médicament ou quasi-médicament pour une administration à des humains ou à des animaux, qui contient la composition selon la revendication 1.

12. Composition solubilisée pulvérulente qui peut être obtenue en soumettant la composition selon la revendication 1 à un séchage par pulvérisation.

13. Procédé pour la solubilisation d'une coenzyme Q réduite et l'inhibition de l'oxydation d'une coenzyme Q réduite représentée par la formule générale (1) où n est un nombre entier de 1 à 12 : 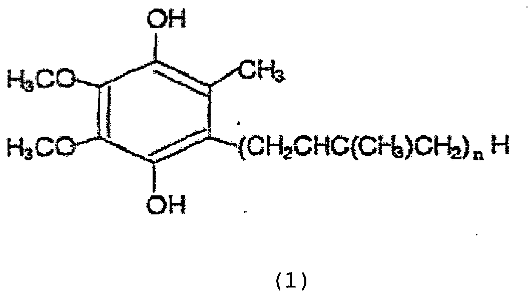 qui comprend le mélange d'une cyclodextrine, d'un solvant polaire et de la coenzyme Q réduite pour donner un composé clathrate.

14. Procédé selon la revendication 13, dans lequel la coenzyme Q oxydée est coprésente, et dans lequel une proportion de la coenzyme Q réduite par rapport à la somme de la coenzyme Q oxydée et la coenzyme Q réduite n'est pas inférieure à 50 % en poids.

15. Procédé selon la revendication 13, dans lequel la coenzyme Q réduite est la coenzyme Q₁₀ réduite.

16. Procédé selon la revendication 13, dans lequel la cyclodextrine est la γ-cyclodextrine.

17. Procédé selon la revendication 13, qui comprend la dissolution de la cyclodextrine dans le solvant polaire, et ensuite le mélange de la coenzyme Q réduite avec la solution obtenue.

18. Procédé selon la revendication 13, dans lequel le solvant polaire est l'eau ou une solution mixte d'eau et d'un alcool.

19. Procédé selon la revendication 13, dans lequel une proportion de la cyclodextrine contenue est de 0,1 à 100 moles pour 1 mole de la coenzyme Q réduite.

20. Procédé selon la revendication 13, dans lequel un antioxydant est co-présent, et dans lequel l'antioxydant est au moins une espèce choisie parmi l'acide citrique, les dérivés de l'acide citrique, la vitamine C, les dérivés de la vitamine C, la vitamine E, les dérivés de la vitamine E, le glutathion, le glutathion réduit, le thiosulfate de sodium, la L-cystéine, la L-carnitine, le lycopène, la riboflavine, les curcuminoïdes et la superoxyde dismutase (SOD).

21. Procédé de production d'une composition contenant une cyclodextrine, un solvant polaire et une coenzyme Q réduite représentée par la formule générale (1) où n est un nombre entier de 1 à 12 : 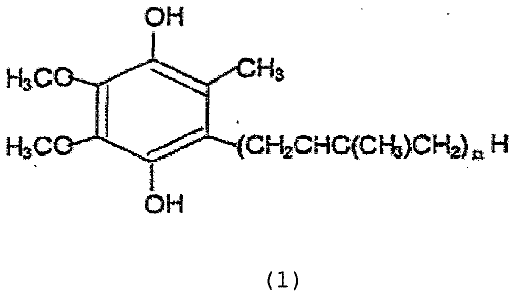 qui comprend le mélange de la coenzyme Q réduite avec la cyclodextrine dans le solvant polaire pour donner un composé clathrate.

22. Procédé selon la revendication 21, qui comprend le mélange de la cyclodextrine, du solvant polaire et de la coenzyme Q réduite, et le séchage par pulvérisation du mélange pour donner une composition pulvérulente.
